# EUROPEAN PATENT SPECIFICATION

(11) **EP 0 442 537 B1**
(45) Date of publication and mention of the grant of the patent: **13.12.1995**
(21) Application number: 91105639.8
(22) Date of filing: 05.04.1986
(51) Int. Cl.: A61K 35/78

(54) **A mental disease therapeutic agent**
Geisteskrankheittherapeutisches Mittel
Agent thérapeutique contre les maladies mentales

(30) Priority: 06.04.1985 JP 73220/85; 26.02.1986 JP 42738/86; 26.02.1986 JP 42739/86; 26.02.1986 JP 42740/86
(43) Date of publication of application: 21.08.1991
(62) Divisional of application: 86104662.1
(73) Proprietor: Nippon Paint Co., Ltd., Osaka-shi Osaka-fu (JP)
(72) Inventor: Yoshikazu, Yamamoto, Neyagawa-shi, Osaka 572 (JP); Ryuzo, Mizuguchi, Yawata-shi, Kyoto-fu (JP); Toshiko, Shibata, Ichikawa-shi, Chiba (JP)
(74) Representative: von Kreisler, Alek, Dipl.-Chem.

(56) References cited:
- EP-A- 1 996 533
- US-A- 4 427 694

## Description

### Field of the invention

The present invention relates to a therapeutic agent for mental disease obtainable from a plant culture cell derived from tissues or cells of plants belonging to Hydrocotyle genus (water-pennywort) and Centella genus.

### Background of the invention

Since the water-pennywort (Hydrocotyle genus) is a perennial herb growing naturally in a yard or field and has a height of less than 10 cm, it is difficult to obtain effective components in large quantities.

Components obtained from plants may be classified based on their functions to a blood coagulation component which indicates a blood coagulating function and a component for improving mental disease which improves mental diseases.

The present invention provides a mental disease therapeutic component, particularly a component for suppressing neurotropic spasm, to produce in large quantities by way of adopting a plant tissue culture technique which has been recently close-uped.

The plant tissue culture can grow plants in very high rates in comparison with natural plants growing in a year or month term. Accordingly, the object substance can be obtained in a short term. The plant tissue culture also is not affected by weather and necessary components can be gathered without lots of labors, and it can be intentionally carried out in an industrial scale.

The culture cell or culture tissue of Hydrocotyle genus and Centella genus, however, has not been reported.

### Summary of the invention

The present invention provides a mental disease therapeutic agent containing a mental disease therapeutic component obtainable by organic solvent extraction from plant cell cultures derived from tissues or cells of a plant belonging to Hydrocotyle or Centella genus.

### Detailed description of the present invention

In the present invention, a plant employed belongs to Hydrocotyle genus or Centella genus. Examples of the plants are Hydrocotyle sibthorpioides, H. Maritima, H. japonica, H. ramiflora, H. nepalensis, Centella asiatica and the like.

The portion to be cultured of the plant is all tissues of the plant belonging to Hydrocotyle genus or Centella genus. A division tissue and a node tissue are preferred because of high propagation rate of the culture cell or culture tissue derived therefrom. By "division tissue" is meant a tissue contributing to growth of plants by way of cell division in the tissue of plants. Preferred are a terminal bud and a lateral bud. By "node tissue" is meant a portion of stalk to which a leaf is attached or being attached, which is contrasted to a tissue between nodes.

The cultured plant cells are plant cells derived from the tissue or cell of plants and artificially cultured in a container. The cultured plant cells include callus tissues, differentiated cultured tissues, a cultured organ tissues and the like. The callus tissues (hereinafter simply referred to "callus") are cultured plant cells lump only consisting of shapeless undifferentiated cells propagated over solid culture medium containing plant hormones or in liquid culture medium containing the plant hormones. The differentiated tissues are cultured plant cells lump composed of tissue having been differentiated (such as a root, a bud or a shoot), and undifferentiated cells. Included are adventive buds (consisting of bud tissues and undifferentiated cells), adventive roots (consisting of root tissues and undifferentiated cells) and cormus cultivated tissues (consisting of shoot tissues and undifferentiated cells). The cultured organs are cultured plant cells lump solely consisting of differentiated tissues which include cultures roots, cultures shoot and the like.

A method for culturing cells is illustrated by adopting Hydrocotyle maritima as an example, but it can apply to the other plants classified as Hydrocotyle genus and Centella genus as well.

A stalk containing petioles and terminal buds of Hydrocotyle maritima is washed with a deionized water and various germs on the surface of it are removed by immersing in a 70% ethanol for 5 to 10 minutes and then in a 10% bleaching powder solution for 5 to 10 minutes followed by removing a remaining sterilizing agent with a sterilized distilled water. The sterilized petiole may be cut with a sterilizing knife to a portion having a suitable size. The terminal bud can also be cut off from the sterilized stalk with the sterilized knife and a sterilized pincette. The cut portion is placed on an inorganic synthetic culture medium preferably containing an auxine and to culture it.

Besides the above petiole or terminal bud, the plant tissue or cell to be cultured may be any other portions, such as a division tissue or a node tissue including a lateral bud, a leaf, a stalk, or a root. The cell obtained by treating the above mentioned tissue or cell, such as a protoplast can also be employed. Further a tissue or cell which has already cultured (hereinafter "culture tissue" or "culture cell") is used.

The culture medium for culturing the plant tissue is known inorganic synthetic agar culture mediums added with a trace organic material, a carbon source, a various natural and an extract together with an auxine and/or a cytokinin. Typical examples of the culture mediums are a White culture medium, a Linsmaier-skoog, a Murashige-skoog culture medium and the like. These culture medium can be changed in its composition.

The trace organic materials include vitamines such as tiamine hydrochloride, pyridoxine hydrochloride, nicotinic acid and the like, amino acids such as glycine, asparagine and the like and heptahydric alcohols such as inositol, sorbitol and the like. The culture medium without adding the trace organic materials may arise good propagation in some cases.

The carbon sources include carbohydrates such as sucrose, glucose, maltose and the like, organic acids such as acetic acids and the like, alcohols such as methanol, glycerol and the like. Preferred are sucrose and glucose because of rapid growing rate of plant cell. The concentration of the carbon sources is within the range of 1 to 10 %w/v, preferably 3 to 5 %w/v.

Examples of the auxines are 2,4-dichlorophenoxyacetic acid (2,4-D), beta-indoleacetic acid (IAA), alpha-naphthaleneacetic acid (NAA), a mixture thereof and the like. The amount of the auxines is not more than 10⁻⁴, preferably not more than 10⁻⁵, more preferably within the range of 10⁻⁷ to 10⁻⁵. The cytokinins includes kinetin, benzyladenine, and a mixture thereof and the like. The amount of the cytokinin is not more than 10⁻⁴ , preferably not more than 10⁻⁵, more preferably within the range of 10⁻⁷ to 10⁻⁵.

Examples of the natural extracts mentioned above are casein hydrolysates (0.01 to 2 %w/v), coconuts milk (5 to 20 %w/v), yeast extracts (0.01 to 2 %w/v), malt extracts (0.01 to 2 %w/v), a mixture thereof and the like. Culture can be carried out bu exposing to light, preferably a light having more than 1,000 lx for more than 16 hours per day.

The resultant culture cells can be either a tissue culture callus or a differentiated tissue depending on the portion of the plant tissue being cultured or a combination of the auxines with the cytokinins. Callus is generally obtained in many conditions. In case where the tissue is a division tissue, especially a terminal bud or a lateral bud, where the auxines are indoleacetic acid or naphthaleneacetic acid, or where the culturing is conducted under light especially having 5,000 lx for more than 16 hours, a differentiated tissue, particularly a cormus culture tissue can be obtained.

The derivation for differentiating the callus to an adventive bud depends on the amount of the auxines and cytokinin or the conditions of light exposure. Preferred condition is set to a combination of 0 to 10⁻⁶ M of the auxine (for example 2,4-D) with 0 to 10⁻⁶ of the cytokinin (for example kinetin) and the light exposure of more than 16 hours at 5,000 lx.

Adventive roots are formed from the callus depending on the amount of the auxines and cytokinins. Preferred amount is a combination of 0 to 10⁻⁶ M of the auxines, typically indole acetic acid or naphthaleneacetic acid and 0 to 10⁻⁶ M of the cytokinin.

The root cultures can be generally derived from the adventive root by cutting off the end portion containing growing point of the adventive root using a knife and placing it on the agar culture medium or into the liquid culture medium to culture. Besides the adventive roots, roots which are developed from a seed under a sterilized condition or which is artificially developed from a plant by inoculating Agrobacterium lisogenase can be employed. As the culture medium, it is preferred that the auxine, preferably indole acetic acid or naphthaleneacetic acid, is added in an amount of 0 to 10⁻⁶ and the cytokinin is added in an amount of 0 to 10⁻⁶. The culture is conducted either in a dark place at 20 to 30 °C for solid culturing medium or in a shaker of 50 to 150 rpm for liquid culture medium, but these are not limited.

A shoot culture can be generally derived from the shoot culture tissues mentioned above as generally described relating to the cultured roots.

The cultured cells can be industrially obtained by culturing and propagating in a similar method of culturing of general microbes. The liquid culture can be classified to a shaking method culture in a shaker and a bubbling method culture by introducing a sterilized air into a container made of glass, metal and the like.

The culture cell obtained above is dried by a freeze drying or an air drying at 60°C for 24 hours or at 110°C for 3 hours to remove water. The dried cultured cells are extracted in acetone by a Soxhlet apparatus, a digestion method, or a maceration method. Instead of acetone, the other organic solvent such as methanol, ethanol and the like may be used for the extraction. Acetone is removed from the extract to obtain a concentrated acetone extract.

It has been found that the plant extract or the plant culture tissue of the present invention, per se, has mental disease therapeutic function. In addition, an extract from plants also has the same function. The obtained mental disease therapeutic component can be used as it is or in the form of a dried powder. The mental disease therapeutic component may be combined with conventional additives. The formulation form of the psychical disease improving agent is limited, including powder, tablet, liquid and the like.

The dosage of the mental disease therapeutic agent is not limited and can vary depending on the purpose or disease conditions. For example, in case of the acetone extract mentioned above, the dosage is 1 to 1,000 mg/day/weight.

The mental disease therapeutic agent of the present invention improves many mental diseases, such as mental uneasiness, melancholia, nicotine toxicosis, alcoholism, morphinomenia, antihypotic toxicosis and the like.

The present invention is illustrated by the following examples, but they are not construed as limiting the present invention.

### Example 1

A 3cm stalk of Hydrocotyle maritima containing terminal buds was washed with water and immersed in a 70% ethanol for 5 minutes and then in a 10% bleaching powder for 10 minutes to sterilize. The stalk was further immersed in a sterilized distilled water for several times to remove a remaining sterilizing agent. The sterilized stalk was cut to a fragment containing a terminal bud having a length of 5 to 1mm with a sterilized knife and a sterilized pincette under a stereomicroscope. The obtained sterilized fragment of Hydrocotyle maritima was placed on a synthetic agar plate having the following composition. The culture medium was made by adding 3% w/v of sucrose, 10⁻⁶ M of alphanaphthaleneacetic acid, 0.1 ppm of tiamine hydrochloride, 0.5 ppm of pyridoxine hydorochloride, 0.5 ppm of nicotinic acid, 2 ppm of glycine and 100 ppm of inositol to an inorganic salt culture medium of Murashige-skoog to adjust to pH 6.0, adding 0.8 %w/v of agar and sterilizing in a conventional manner.

The fragment placed on the culture medium was cultured at the culture temperature of 25°C under the light f 5,000 lx. After three weeks, shoot cultured tissues were developed from the fragment. The shoot cultured tissues were divided to two portions after one month and transferred to other culture mediums having the same composition to continue culturing at 25°C. The same treatment was repeated every 2 weeks to obtain stable shoot cultured tissues.

### Example 2

A stalk of Hydrocotyle maritima containing node tissues was washed with water and immersed in a 70% ethanol for 5 minutes and then in a 10% bleaching powder for 10 minutes to sterilize. The stalk was further immersed in a sterilized distilled water for several times to remove a remaining sterilizing agent. The sterilized stalk was cut to a fragment containing a node tissue having a length of 0.5 to 1mm with a sterilized knife. The obtained sterilized fragment of Hydrocotyle maritima was placed on a synthetic agar plate having the following composition. The culture medium was made by adding 3% w/v of sucrose, 10⁻⁵ M of kinetin, 10⁻⁶ of 2,4 dichlorophenoxyacetic acid, 0.1ppm of tiamine hydrochloride, 0.5ppm of pyridoxine hydorochloride, 0.5ppm of nicotinic acid, 2ppm of glycine and 100ppm of inositol to an inorganic salt culture medium of Murashige-skoog to adjust to pH6.0, adding 0.8%w/v of agar and sterilizing in a conventional manner.

The fragment placed on the culture medium was cultured at the culture temperature of 25°C. After about one week, a light yellow callus was developed from near the cut portion. The grown-up callus was divided to two portions after one month and transferred to other culture mediums having the same composition to continue culturing at 25°C. The same treatment was repeated every 2 or 3 weeks to obtain a stable callus.

The propagation ratio of the callus in two weeks is shown in Table 1 infra.

The callus of Hydrocotyle maritima was taken from the solid culture medium and dried at 60°C for 24 hours to obtain a dried callus. The dried calluses were ground by a mortar and extracted by acetone using a Soxhlet extractor for 8 hours for three times. The acetone was removed by distillation to obtain 1.5g of an acetone extract. The extract had blood coagulation function.

Bananas containing 5ml of a triolein in which 200mg of the extract was dissolved was eaten to a monkey (2.1Kg) of alcoholism for seven days. The alcoholism symptoms, such as shaking and stiffening of muscle were improved.

### Example 3

A callus of Centella asiatica was formed by culturing as generally described in Example 1 with the exception that Centella asiatica was employed instead of Hydrocotyle maritima.

The propagation ratio of the callus in two weeks is shown in Table 1 infra.

The acetone extract of the callus showed improving function of alcoholism.

### Example 4

A callus derived from internode tissue of Hydrocotyle maritima was formed by culturing as generally described in Example 1 with the exception that the internode tissue was employed instead of the node tissue.

The propagation ratio of the callus in two weeks is shown in Table 1 infra.

### Example 4

A callus derived from an internode tissue of Centella asiatica was formed by culturing as generally described in Example 3 with the exception that the internode tissue was employed instead of the node tissue.

The propagation ratio of the callus in two weeks is shown in Table 1 infra.

**Table 1**

| Example | Propagation ration in two weeks |
|---|---|
| 7 | 6.8 |
| 8 | 6.4 |
| 9 | 3.1 |
| 10 | 2.5 |

### Example 5

A petiole of Hydrocotyle maritima was washed with water and immersed in a 70% ethanol for 5 minutes and then in a 10% bleaching powder for 10 minutes to sterilize. The petiole was further immersed in a sterilized distilled water for several times to remove a remaining sterilizing agent. The sterilized petiole was cut to a fragment having a length of 0.5 to 1mm with a sterilized knife. The obtained sterilized fragment of Hydrocotyle maritima was placed on a synthetic agar plate medium having the following composition. The culture medium was made by adding 3% w/v of cane sugar, 10⁻⁵ of kinetin, 10⁻⁶ M of 2,4-dichlorophenoxyacetic acid. 0.1ppm of tiamine hydrochloride, 0.5ppm of pyridoxine hydorochloride, 0.5ppm of nicotinic acid, 2ppm of glycine and 100ppm of inositol to an inorganic salt culture medium of Murashige-skoog to adjust to pH6.0, adding 0.8%w/v of agar and sterilizing in a conventional manner.

The fragment placed on the culture medium was cultured at the culture temperature of 25°C. After one week, a light yellow callus was developed from the fragment. The grown-up callus was divided to several portions after one month and transferred to other culture mediums having the same composition to continue culturing at 25°C. The same treatment was repeated every 4 to 6 weeks to obtain a stable callus.

The calluses of H. maritima were taken from the solid culture medium and dried at 60°C for 24 hours to obtain a dried calluses of 10g. The dried calluses were ground in a mortar and extracted by acetone using a Soxhlet extractor for 8 hours for three times. The obtained extract was subjected to a distillation for removing acetone to obtain a concentrated acetone extract of 1.5g.

### Example 6

Four hundred gram of the calluses obtained from Example 5 was dispersed in a liquid culture medium of 10 liters in a jar of 14 liters and cultured for ten days at agitating rate of 50rpm, air supply rate of 5 l/min and temperature of 25 °C. The culture medium employed was MS medium with 3% sucrose (w/v), 10⁻⁵ kinetin, 10⁻⁶M 2,4-D, 0.1ppm thiamine·HCl, 0.5ppm nicotinic acid, 2ppm of glycine and 100ppm of inositol, 0.8% (w/v) agar, pH6.0. After ten days, calluses of about 4 kg was formed. Acetone concentrated extract of 20 g was obtained as generally described in Example 5.

### Example 7

The callus of Hydrocotyle maritima obtained in Example 5 was transferred into a agar-plate having the following composition. The culture medium was formed by adding 3% w/v of sucrose, 10⁻⁶ of indole acetic acid, 0.1ppm of tiamine hydrochloride, 0.5ppm of pyridoxine hydorochloride, 0.5ppm of nicotinic acid, 2ppm of glycine and 100ppm of inositol to an inorganic salt culture medium of Murashige-skoog to adjust to pH6.0, adding 0.8%w/v of agar and sterilizing in a conventional manner. The culturing was conducted at 25 °C and 120 rpm to form an adventive root after two weeks.

Acetone extract was obtained from the differentiated adventive root by treating as generally described in Example 5.

### Example 8 to 12

Acetone extract was obtained by treating as generally described in Example 7 with the exception that Hydrocotyle sibthorpioides, H. nepalensis, H. ramiflora, H. japonica or Centella asiatica was respectively employed instead of Hydrocotyle maritima.

### Example 13

The end portion of the adventive roots obtained by the process of Example 7 was sterilely cut off and transferred to the same culture medium as Example 7 to form cultured roots having propagating ability of prolonging and dividing.

Acetone extract was obtained from the cultured roots by treating as generally described in Example 41.

### Example 14 to 18

Acetone extract was obtained by treating as generally described in Example 13 with the exception that Hydrocotyle sibthorpioides, H. nepalensis, H. ramiflora, H. japonica or Centella asiatica was respectively employed instead of Hydrocotyle maritima.

### Example 19

A 3cm stalk of Hydrocotyle maritima containing terminal buds was washed with water and immersed in a 70% ethanol for 5 minutes and then in a 10 % bleaching powder for 10 minutes to sterilize. The stalk was further immersed in a sterilized distilled water for several times to remove a remaining sterilizing agent. The sterilized stalk was cut to a fragment containing a terminal bud having a length of 5 to 1mm with a sterilized knife and a sterilized pincette under a stereomicroscope. The obtained sterilized fragment of Hydrocotyle maritima was placed on a synthetic agar plate having the following composition. The culture medium was made by adding 3 % w/v of sucrose, 10⁻⁶ M of alpha-naphthaleneacetic acid, 0.1ppm of tiamine hydrochloride, 0.5ppm of pyridoxine hydorochloride, 0.5ppm of nicotinic acid, 2ppm of glycine and 100ppm of inositol to an inorganic salt culture medium of Murashige-skoog to adjust to pH6.0, adding 0.8%w/v of agar and sterilizing in a conventional manner.

The fragment placed on the culture medium was cultured at the culture temperature of 25°C under the light of 5,000 lx. After three weeks, shoot cultured tissues were developed from the fragment. The grown-up shoot cultured tissues were divided to two portions after one month and transferred to other culture mediums having the same composition to continue culturing at 25°C. The same treatment was repeated every 2 weeks to obtain stable shoot cultured tissues.

The end portion of the shoot was sterilely cut off and transferred to the liquid culture medium having the same composition mentioned above. Culturing was conducted at 120 rpm and 25 °C under the light of 5,000 lx to obtain cultured shoot having propagating ability of prolonging and branching.

Acetone extract was obtained from cultured shoot as generally described in Example 5.

### Example 20

Acetone extract was obtained by treating as generally described in Example 19 with the exception that Hydrocotyle sibthorpioides, H. nepalensis, H. ramiflora, H. japonica or Centella asiatica was respectively employed instead of Hydrocotyle maritima.

### Example 21

Bananas containing 5ml of a triolein in which 200mg of the acetone extract obtained in Example 5 was dissolved was eaten to a monkey (2 Kg) of alcoholism for seven days. The alcoholism symptons, such as shaking and stiffening of muscle, were improved.

### Example 22

One hundred gram of whole plant of H. maritima was dried for 24 hours at 60 °C to obtain 5.5 g of a dried plant. The dried plant was ground by a mortar and extracted by acetone using a Soxhlet extractor for 8 hours for three times. Acetone was distilled out from the acetone extract to obtain an acetone concentrated extract (1.1 g).

The test was conducted as described in Example 21 using the obtained acetone concentrated extract. The result showed similar improving effect of alcoholism symptons.

### Example 23

Acetone concentrated extract (1.4 g) was obtained by treating as generally described in Example 5 with the exception that the callus of H. maritima was employed as a plant tissue. The dried calluses were 10 g.

The test was conducted as described in Example 21 using the obtained acetone concentrated extract. The result showed similar improving effect of alcoholism symptons.

### Example 24

Acetone concentrated extract (1.0 g) was obtained by treating as generally described in Example 130 with the exception that whole plant of H. maritima was employed as a plant tissue. The dried calluses were 60 g.

The test was conducted as described in Example 21 using the obtained acetone concentrated extract. The result showed similar improving effect of alcoholism symptons.

### Example 25

Acetone concentrated extract (1.5 g) was obtained by treating as generally described in Example 5 with the exception that the callus of H. japonica was employed as a plant tissue. The dried calluses were 10 g.

The test was conducted as described in Example 129 using the obtained acetone concentrated extract. The result showed similar improving effect of alcoholism symptons.

### Example 26

Acetone concentrated extract (0.8 g) was obtained by treating as generally described in Example 22 with the exception that whole plant of H. japonica was employed as a plant tissue. The dried calluses were 5.1 g.

The test was conducted as described in Example 21 using the obtained acetone concentrated extract. The result showed similar improving effect of alcoholism symptons.

### Example 27

Acetone concentrated extract (1.3 g) was obtained by treating as generally described in Example 5 with the exception that the callus of H. ramiflora was employed as a plant tissue. The dried calluses were 10 g.

The test was conducted as described in Example 21 using the obtained acetone concentrated extract. The result showed similar improving effect of alcoholism symptons.

### Example 28

Acetone concentrated extract (0.9 g) was obtained by treating as generally described in Example 22 with the exception that whole plant of H. ramiflora was employed as a plant tissue. The dried calluses were 6.2 g.

The test was conducted as described in Example 21 using the obtained acetone concentrated extract. The result showed similar improving effect of alcoholism symptons.

### Example 29

Acetone concentrated extract (1.5 g) was obtained by treating as generally described in Example 5 with the exception that the callus of H. nepalensis was employed as a plant tissue. The dried calluses were 10 g.

The test was conducted as described in Example 21 using the obtained acetone concentrated extract. The result showed similar improving effect of alcoholism symptons.

### Example 30

Acetone concentrated extract (0.8 g) was obtained by treating as generally described in Example 22 with the exception that whole plant of H. nepalensis was employed as a plant tissue. The dried calluses were 5.2 g.

The test was conducted as described in Example 21 using the obtained acetone concentrated extract. The result showed similar improving effect of alcoholism symptons.

### Example 31

Acetone concentrated extract (1.4 g) was obtained by treating as generally described in Example 5 with the exception that the callus of Centella asiatica was employed as a plant tissue. The dried calluses were 10 g.

The test was conducted as described in Example 21 using the obtained acetone concentrated extract. The result showed similar improving effect of alcoholism symptons.

### Example 32

Acetone concentrated extract (1.0 g) was obtained by treating as generally described in Example 22 with the exception that whole plant of C. asiatica was employed as a plant tissue. The dried calluses were 5.5 g.

The test was conducted as described in Example 21 using the obtained acetone concentrated extract. The result showed similar improving effect of alcoholism symptons.

## Claims

1. A mental disease therapeutic agent comprising a mental disease therapeutic component obtainable by organic solvent extraction from a cultured cells derived from tissues or cells of a plant belonging to Hydrocotyle genus or Centella genus.

2. The method for preparing the mental disease therapeutic component of claim 1 comprising extracting cultured cells derived from tissues or cells of a plant belonging to Hydrocotyle genus or Centella genus.

## Patentansprüche

1. Therapeutikum für Geisteskrankheiten, das eine bei Geisteskrankheiten wirksame therapeutische Komponente umfaßt, die durch Extraktion mit einem organischen Lösungsmittel aus kultivierten Zellen erhältlich ist, welche aus Geweben oder Zellen einer Pflanze abgeleitet sind, die zur Gattung *Hydrocotyle* oder zur Gattung *Centella* gehört.

2. Verfahren zur Herstellung der bei Geisteskrankheiten wirksamen therapeutischen Komponente gemäß Anspruch 1, umfassend das Extrahieren kultivierter Zellen, die aus Geweben oder Zellen einer Pflanze abgeleitet sind, die zur Gattung *Hydrocotyle* oder zur Gattung *Centella* gehört.

## Revendications

1. Agent thérapeutique contre les maladies mentales comprenant un composé thérapeutique contre les maladies mentales pouvant être obtenues par extraction par un solvant organique à partir de cellules cultivées dérivées de tissus ou de cellules d'une plante appartenant aux genres Hydrocotyle ou Centella.

2. Méthode de préparation du composé thérapeutique contre les maladies mentales de la revendication 1 comprenant l'extraction de cellules cultivées dérivées de tissus ou de cellules d'une plante appartenant aux genres Hydrocotyle ou Centella.
